(19) Europäisches Patentamt
European Patent Office
Office européen des brevets

(11) EP 1 872 783 B1

(12) **EUROPEAN PATENT SPECIFICATION**

(45) Date of publication and mention of the grant of the patent:
**28.12.2011 Bulletin 2011/52**

(21) Application number: **06732190.1**

(22) Date of filing: **21.04.2006**

(51) Int Cl.:
***A61K 31/41*** *(2006.01)* ***A61P 27/02*** *(2006.01)*

(86) International application number:
**PCT/JP2006/308382**

(87) International publication number:
**WO 2006/115185 (02.11.2006 Gazette 2006/44)**

# (54) THERAPEUTIC AGENT FOR CORNEAL/CONJUNCTIVAL DISORDER

THERAPEUTISCHES MITTEL GEGEN HORNHAUT/BINDEHAUT-ERKRANKUNGEN

AGENT THÉRAPEUTIQUE POUR UN TROUBLE CORNÉEN/CONJONCTIVAL

(84) Designated Contracting States:
**AT BE BG CH CY CZ DE DK EE ES FI FR GB GR HU IE IS IT LI LT LU LV MC NL PL PT RO SE SI SK TR**

(30) Priority: **21.04.2005 JP 2005123791**

(43) Date of publication of application:
**02.01.2008 Bulletin 2008/01**

(73) Proprietor: **SANTEN PHARMACEUTICAL CO., LTD.**
**Higashiyodogawa-ku,**
**Osaka-shi,**
**Osaka 533-8651 (JP)**

(72) Inventors:
- **SHIBAGAKI, Keiichi,**
  **SANTEN PHARMACEUTICAL CO. LTD**
  **Ikoma-shi, Nara 6300101 (JP)**
- **HIRAI, Shin-ichiro,**
  **SANTEN PHARMACEUTICAL CO. LTD**
  **Ikoma-shi, Nara 6300101 (JP)**
- **NAKAMURA, Masatsugu,**
  **SANTEN PHARMACEUTICAL CO LTD**
  **Ikoma-shi, Nara 6300101 (JP)**

(74) Representative: **Müller-Boré & Partner**
**Patentanwälte**
**Grafinger Straße 2**
**81671 München (DE)**

(56) References cited:

| | |
|---|---|
| **EP-A- 1 197 223** | **JP-A- 03 027 362** |
| **JP-A- 03 063 264** | **JP-A- 08 067 674** |
| **JP-A- 09 508 641** | **JP-A- 10 218 792** |
| **JP-A- 2000 143 650** | **JP-A- 2001 055 329** |

- **KIKUCHI T ET AL: "Mechanism of permeability-enhancing effect of EDTA and boric acid on the corneal penetration of 4-[1-hydroxy-1-methylethyl]-2-propyl-1-[4- [2-[tetrazole- 5-yl] phenyl]phenyl] methylimidazole-5-carboxylic acid monohydrate (CS-088)" INTERNATIONAL JOURNAL OF PHARMACEUTICS 20050811 NL, vol. 299, no. 1-2, 11 August 2005 (2005-08-11), pages 107-114, XP002530133 ISSN: 0378-5173**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 872 783 B1

## Description

Technical Field

The present invention relates to a therapeutic agent for a keratoconjunctival disorder such as dry eyes, corneal ulcer, keratitis, conjunctivitis, superficial punctate keratopathy, corneal epithelial defects, conjunctival epithelial defects, keratoconjunctivitis sicca, superior limbic keratoconjunctivitis or filamentary keratitis, comprising as an active ingredient, a biphenylmethyl derivative or a salt thereof.

Background Art

Cornea is a transparent avascular tissue having a diameter of about 1 cm and a thickness of about 1 mm, while conjunctiva is a mucosal membrane covering the eyeball surface posterior to the corneal margin, and the back face of the eyelid. It is known that when the cornea or the conjunctiva is damaged, the visual function is significantly affected. Keratoconjunctival disorders caused due to a variety of diseases such as corneal ulcer, keratitis, conjunctivitis, dry eyes and the like may adversely affect normal architecture of corneal epithelium or conjunctival epithelium, and as a result, they may impair structures and functions of the corneal stroma and corneal endothelium. In these years, with the development of cell biology, factors participating in cell proliferation, migration, adhesion, extension, differentiation and the like have been elucidated, and it has been reported that these factors play important roles in repair of keratoconjunctival disorders (Japanese Review of Clinical Ophthalmology, 46, 738-743 (1992), Ophthalmic Surgery, 5, 719-727 (1992)).

On the other hand, it is disclosed that a biphenylmethyl derivative, which is an active ingredient of the present invention, inhibits the action of angiotensin II. and is useful as a therapeutic agent for cardiovascular diseases such as hypertension and heart failure (Japanese patent No. 2709225, Japanese patent No. 2868313, JP-B-5-29351, etc.).

However, there is no report of study on a pharmacological action of such a biphenylmethyl derivative on an eye disease, particularly a keratoconjunctival disorder, and also there is no suggestion as to what type of biphenylmethyl derivative with what basic chemical structure is useful for a keratoconjunctival disorder.

Disclosure of the invention

Problem to be Solved

Accordingly, it is a very interesting subject to research a compound useful as a therapeutic agent for a keratoconjunctival disorder among these numerous known biphenylmethyl derivatives.

Means of Solving Problems

The present inventors have made intensive studies on effects of biphenylmethyl derivatives in treatment of a corneal disorder, and as a result, they found that a biphenylmethyl derivative having a specific basic chemical structure exhibits an excellent improving effect on a corneal disorder in a test for therapeutic effect using corneal disorder models, and thus the present invention has been accomplished.

That is, the present invention is directed to:

(1) a therapeutic agent for a keratoconjunctival disorder comprising as an active ingredient a compound represented by the following general formula (1) or a salt thereof:

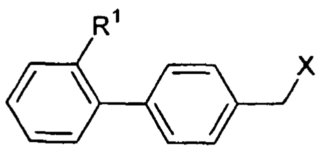

(1)

(wherein X represents

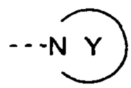 or 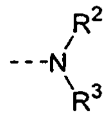 ,

the ring Y represents a substituted or unsubstituted nitrogen-containing heterocyclic ring,

$R^1$ represents a carboxy group or a substituted or unsubstituted nitrogen-containing 5-membered heterocyclic ring, and

$R^2$ and $R^3$ may be the same or different and represent a hydrogen atom, a substituted or unsubstituted alkyl group or an unsubstituted alkylcarbonyl group);

(2) the therapeutic agent for a keratoconjunctival disorder according to the above (1), wherein in the general formula (1),

X represents

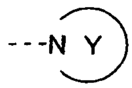 ,

the ring Y represents

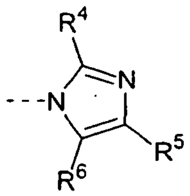 , 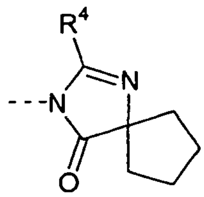 or 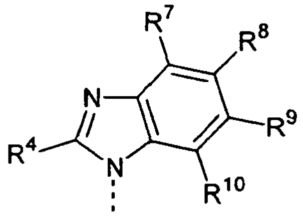 ,

$R^1$ represents a carboxy group,

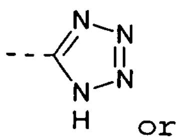 or 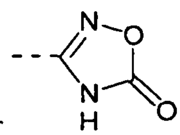 ,

$R^4$ represents a hydrogen atom, a hydroxy group, an alkoxy group or an alkyl group,

$R^5$ and $R^6$ may be the same or different and represent a halogen atom, a hydroxyalkyl group or an alkoxyalkyl group, and

$R^7$, $R^8$, $R^9$ and $R^{10}$ may be the same or different and represent a hydrogen atom, a hydroxy group, an alkoxy group or a carboxy group or an ester thereof;

(3) the therapeutic agent for a keratoconjunctival disorder according to the above (1) or (2), wherein in the general formula (1),

X represents

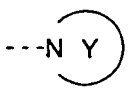 ,

the ring Y represents

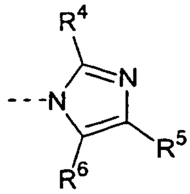

,

$R^1$ represents a carboxy group or

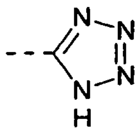

,

$R^4$ represents an alkoxy group or an alkyl group, $R^5$ represents a halogen atom or a hydroxyalkyl group, and $R^6$ represents a halogen atom, a hydroxyalkyl group or a carboxy group or an ester thereof;
(4) the therapeutic agent for a keratoconjunctival disorder according to the above (1) or (2), wherein in the general formula (1),
X represents

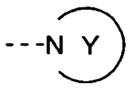

,

the ring Y represents

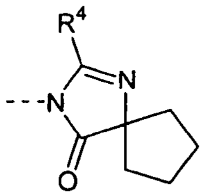

,

$R^1$ represents

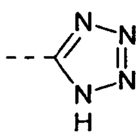

,

and
$R^4$ represents an alkyl group;
(5) the therapeutic agent for a keratoconjunctival disorder according to the above (1) or (2), wherein in the general formula (1),
X represents

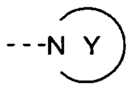,

the ring Y represents

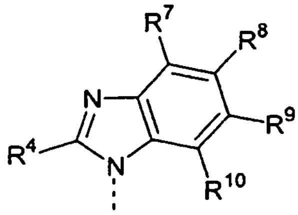,

$R^1$ represents a carboxy group or

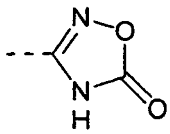,

$R^4$ represents an alkoxy group or an alkyl group,
$R^7$ represents a hydrogen atom or an alkyl group,
$R^8$ represents a hydrogen atom,
$R^9$ represents a hydrogen atom or

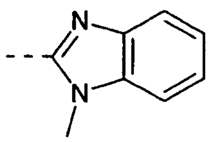,

and
$R^{10}$ represents a hydrogen atom or a carboxy group or an ester thereof;
(6) the therapeutic agent for a keratoconjunctival disorder according to the above (3) or (5), wherein the ester of a carboxy group is

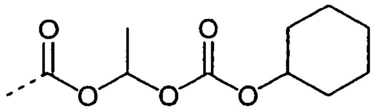 or 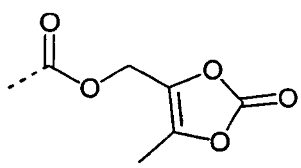;

(7) the therapeutic agent for a keratoconjunctival disorder according to the above (1), wherein in the general formula (1),
X represents

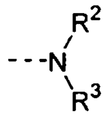 ,

$R^1$ represents

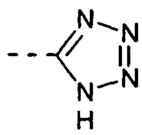 ,

and

$R^2$ and $R^3$ may be the same or different and represent a hydrogen atom, a carboxyalkyl group or an alkylcarbonyl group;

(8) the therapeutic agent for a keratoconjunctival disorder according to the above (1) or (7), wherein in the general formula (1),

X represents

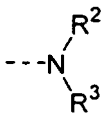 ,

$R^1$ represents

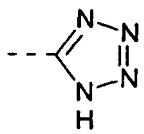 ,

$R^2$ represents a carboxyalkyl group, and

$R^3$ represents an alkylcarbonyl group;

(9) the therapeutic agent for a keratoconjunctival disorder according to the above (1), wherein 4'-[[4-methyl-6-(1-methylbenzimidazol-2-yl)-2-n-propylbenzimidazol-1-yl]methyl]-1,1'-biphenyl-2-carboxylic acid, 2- n-butyl-4-spirocyclopentane-1-[[2'-(1H-tetrazol-5-yl)-1,1'-biphenyl-4-yl]methyl]-2-imidazolin-5-one, 2-butyl -4-chloro-5-hydroxymethyl-1-[[2'-(1H-tetrazol-5-yl)-1,1'-biphenyl-4-yl]methyl]imidazole, 2-ethoxy-1-[[2'- (1H-tetrazol-5-yl)-1,1'-biphenyl-4-yl]methyl]-1H-benzimidazol-7-carboxylic acid, 1-(cyclohexyloxycarbonyloxy) ethyl 2-ethoxy-1-[[2'-(1H-tetrazol-5-yl)-1,1'- biphenyl-4-yl]methyl]-1H-benzimidazol-7-carboxylate, N-[[2'-(1H-tetrazol-5-yl)-1,1'-biphenyl-4-yl]methyl]-N-valeryl-L-valine, 4-(1-hydroxy-1-methylethyl)-2-n- propyl-1-[[2'-(1H-tetrazol-5-yl)-1,1'-biphenyl-4-yl]methyl]imidazol-5-carboxylic acid or (5-methyl-2-oxo- 1,3-dioxol-4-yl)methyl 4-(1-hydroxy-1-methylethyl)-2- n-propyl-1-[[2'-(1H-tetrazol-5-yl)-1,1'-biphenyl-4-yl]methyl]imidazol-5-carboxylate, or a salt thereof is contained as an active ingredient;

(10) the therapeutic agent according to any one of the above (1) to (9), wherein the keratoconjunctival disorder is dry eyes, corneal ulcer, keratitis, conjunctivitis, superficial punctate keratopathy, corneal epithelial defects, conjunctival epithelial defects, keratoconjunctivitis sicca, superior limbic keratoconjunctivitis or filamentary keratitis; and

(11) the therapeutic agent for a keratoconjunctival disorder according to any one of the above (1) to (10), wherein the dosage form is an eye drop or an ophthalmic ointment.

The groups defined in the biphenylmethyl derivative having a basic chemical structure represented by the above general formula (1) of the present invention (hereinafter referred to as the "present compound") will be described in further detail. The halogen refers to fluorine, chlorine, bromine or iodine. The alkyl refers to straight-chain or branched

alkyl having 1 to 6 carbon atoms such as methyl, ethyl, n-propyl, isopropyl, n-butyl, isobutyl, sec-butyl, tert-butyl, n-pentyl, isopentyl, neopentyl, n-hexyl and isohexyl. The alkoxy refers to straight-chain or branched alkoxy having 1 to 6 carbon atoms such as methoxy, ethoxy, n-propoxy, isopropoxy, n-butoxy, tert-butoxy, n-pentyloxy and n-hexyloxy.

The substituted or unsubstituted nitrogen-containing heterocyclic ring refers to a nitrogen-containing heterocyclic ring which may have a substituent, and examples of the nitrogen-containing heterocyclic ring include pyridine, pyrimidine, pyrrole, imidazole, pyrazole, triazole, tetrazole, triazine, tetrahydroquinoline, tetrahydroisoquinoline, indole, quinoline, phenanthridine, benzimidazole and the like. The substituted or unsubstituted nitrogen-containing 5-membered heterocyclic ring refers to a nitrogen-containing 5-membered heterocyclic ring which may have a substituent, and examples of the nitrogen-containing 5-membered heterocyclic ring include pyrrole, imidazole, pyrazole, triazole, tetrazole, triazine and the like.

Further, the ester of a carboxy group refers to an ester composed of a carboxy group and a substituted or unsubstituted alkyl alcohol, a substituted or unsubstituted aryl alcohol or the like. Specific examples of the alkyl alcohol include methanol, ethanol, propanol, butanol and the like, and specific examples of the aryl alcohol include phenol, naphthol and the like.

Preferred examples of $R^1$ in the general formula [1] include a carboxy group,

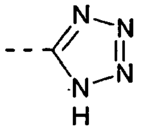 , and 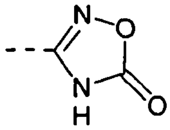

Preferred examples of $R^2$ and $R^3$ include an n-butylcarbonyl group and a 1-carboxy-2-methyl-propyl group.

Preferred examples of $R^4$ include an n-propyl group, an n-butyl group and an ethoxy group.

Preferred examples of $R^5$ include a chlorine atom and a 1-hydroxy-1-methylethyl group.

Preferred examples of $R^6$ include a carboxy group, a hydroxymethyl group, and

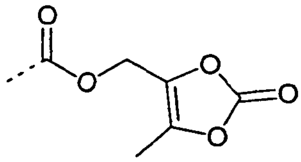 .

Preferred examples of $R^7$ include a hydrogen atom and a methyl group.

Preferred examples of $R^8$ include a hydrogen atom.

Preferred examples of $R^9$ include a hydrogen atom, and

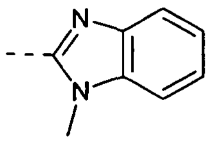 .

Preferred examples of $R^{10}$ include a hydrogen atom, a carboxy group, and

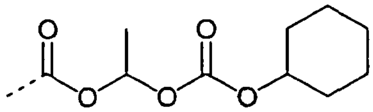 .

Specific preferred examples of the present compound include 4'-[[4-methyl-6-(1-methylbenzimidazol-2-yl)-2-n-propylbenzimidazol-1-yl]methyl]-1,1'-biphenyl-2-carboxylic acid, 2-n-butyl-4-spirocyclopentane-1-[[2'-(1H- tetrazol-5-yl)-1,1'-biphenyl-4-yl]methyl]-2-imidazolin-5-one, 2-butyl-4-chloro-5-hydroxymethyl-1-[[2'-(1H- tetrazol-5-yl)-1,1'-biphenyl-4-yl]methyl]imidazole, 2-ethoxy-1-[[2'-(1H-tetrazol-5-yl)-1,1'-biphenyl-4-yl]methyl]-1H-benzimidazol-7-carboxylic acid, 1-(cyclohexyloxycarbonyloxy)ethyl 2-ethoxy-1-[[2'-(1H- tetrazol-5-yl)-1,1'-biphenyl-4-yl]methyl]-1H-benzimidazol-7-carboxylate, N-[[2'-(1H-tetrazol-5-yl)-1,1'- biphenyl-4-yl]methyl]-N-valeryl-L-valine, 4-(1- hydroxy-1-methylethyl)-2-n-propyl-1-[[2'-(1H-tetrazol-5-yl)-1,1'-biphenyl-4-yl]methyl]imidazol-5-carboxylic acid, (5-methyl-2-oxo-1,3-dioxol-4-yl)methyl 4-(1- hydroxy-1-methylethyl)-2-n-propyl-1-[[2'-(1H-tetrazol-5-yl)-1,1'-biphenyl-4-yl]methyl]imidazol-5-carboxylate, 2-ethoxy-1-[[2'-(5-oxo-2H-1,2,4-oxadiazol-3-yl)- 1,1'-biphenyl-4-yl]methyl]benzimidazol-7-carboxylic acid, 2-butyl-4-chloro-1-[[2'-(1H-tetrazol-5-yl)-1,1'- biphenyl-4-yl]methyl]imidazol-5-carboxylic acid and the like.

The salt of the present compound is not particularly limited as long as it is a pharmaceutically acceptable salt, and examples thereof include sodium salts, potassium salts, lithium salts, calcium salts, magnesium salts, salts with an inorganic acid such as hydrochloric acid, nitric acid or sulfuric acid, salts with an organic acid such as acetic acid, fumaric acid, maleic acid, succinic acid or tartaric acid, and the like. Quaternary ammonium salts are also included in the salt according to the present invention. Preferred salts are sodium salts and potassium salts. The present compound may be in a form of a hydrate or a solvate. Further, optical isomers, geometric isomers, tautomers, polymorphisms and the like of the present compound are also included in the scope of the present invention.

The present compound can be produced based on the method described in Japanese patent No. 2709225, Japanese patent No. 2868313, JP-B-5-29351, Japanese patent No. 2853611, Japanese patent No. 2514282, Japanese patent No. 2749458, JP-B-7-25738, Japanese patent No. 2645962 or Japanese patent No. 3465215.

The keratoconjunctival disorder as used herein means the state of damaged cornea and/or conjunctiva due to various factors, and examples thereof include dry eyes, corneal ulcer, keratitis, conjunctivitis, superficial punctate keratopathy, corneal epithelial defects, conjunctival epithelial defects, keratoconjunctivitis sicca, superior limbic keratoconjunctivitis, filamentary keratitis and the like.

The therapeutic agent for a keratoconjunctival disorder of the present invention may be administered either orally or parenterally.

Examples of the dosage form include eye drops, ophthalmic ointments, injections, tablets, capsules, granules, powders and the like. In particular, eye drops are preferred. These can be prepared using any of generally used techniques. For example, the eye drops can be prepared using a tonicity agent such as sodium chloride or concentrated glycerin, a buffer such as sodium phosphate or sodium acetate, a surfactant such as polyoxyethylene sorbitan monooleate, polyoxyl 40 stearate or polyoxyethylene hydrogenated castor oil, a stabilizer such as sodium citrate or sodium edetate, a preservative such as benzalkonium chloride or paraben as needed. The pH of the eye drops is permitted as long as it falls within the range that is acceptable as an ophthalmic preparation, but is preferably in the range of from 4 to 8.

The ophthalmic ointments can be prepared with a generally used base such as white soft paraffin or liquid paraffin. Also, oral preparations such as tablets, capsules, granules and powders can be prepared by adding an extender such as lactose, crystalline cellulose, starch or vegetable oil, a lubricant such as magnesium stearate or talc, a binder such as hydroxypropyl cellulose or polyvinyl pyrrolidone, a disintegrant such as carboxymethyl cellulose calcium or low-substituted hydroxypropylmethyl cellulose, a coating agent such as hydroxypropylmethyl cellulose, macrogol or a silicone resin, a film forming agent such as gelatin film, and the like, as needed.

The present invention also relates to a method for treating a keratoconjunctival disorder comprising administering to a patient a therapeutically effective amount of a compound represented by the general formula (1) or a salt thereof.

The dose of the present compound can be properly selected depending on the symptoms, age, dosage form and the like. In the case of an eye drop, it may be instilled once to several times a day at a concentration of from 0.00001 to 5% (w/v), preferably from 0.001 to 3% (w/v). In the case of an oral preparation, it may be administered once or divided into several times at a dose of generally from 0.1 to 5000 mg per day, preferably from 1 to 1000 mg per day.

## Advantage of the Invention

As will be described below, when a test for a therapeutic effect on a corneal damage was carried out, any of the present compounds exhibited an excellent improving effect in corneal disorder models. Therefore the present compounds are useful as a therapeutic agent for a keratoconjunctival disorder such as dry eyes, corneal ulcer, keratitis, conjunctivitis, superficial punctate keratopathy, corneal epithelial defects, conjunctival epithelial defects, keratoconjunctivitis sicca, superior limbic keratoconjunctivitis, filamentary keratitis and so on.

## Best Mode for Carrying Out the Invention

Hereinafter, results of a pharmacological test and preparation examples will be shown, however, these examples are for understanding the present invention well, and are not meant to limit the scope of the present invention.

[Pharmacological Test]

Test For Therapeutic Effect On Corneal Damage

Using male SD rats, corneal disorder models were produced in accordance with the method of Fujihara et al. (Invest. Ophthalmol. Vis. Sci. 42 (1): 96-100 (2001)). After the production of the corneal disorder models, the corneal damage score was evaluated in accordance with the method of Murakami et al. (Journal of the eye 21 (1) : 87-90 (2004)), and the improvement ratio of a corneal damage after instillation was obtained.

(Test Method)

Male SD rats were systemically anesthetized by an administration of Nembutal. Subsequently the exorbital lacrimal gland of each rat was removed and a corneal damage was induced over a period of 2 months.

Then, 4'-[[4-methyl-6-(1-methylbenzimidazol-2-yl) - 2-n-propylbenzimidazol-1-yl]methyl]-1,1'-biphenyl-2-carboxylic acid (hereinafter referred to as "Compound A"), 2-n-butyl-4-spirocyclopentane-1-[[2'-(1H-tetrazol-5-yl)-1,1'-biphenyl-4-yl]methyl]-2-imidazolin-5-one (hereinafter referred to as "Compound B"), 2-butyl-4- chloro-5-hydroxymethyl-1-[[2'-(1H-tetrazol-5-yl)-1,1'-biphenyl-4-yl]methyl]imidazole monopotassium salt (hereinafter referred to as "Compound C"), 2-ethoxy-1- [[2'-(1H-tetrazol-5-yl)-1,1'-biphenyl-4-yl]methyl]-1H-benzimidazol-7-carboxylic acid (hereinafter referred to as "Compound D"), N-[[2'-(1H-tetrazol-5-yl)-1,1'- biphenyl-4-yl]methyl]-N-valeryl-L-valine (hereinafter referred to as "Compound E"), or 4-(1-hydroxy-1- methylethyl)-2-n-propyl-1-[[2'-(1H-tetrazol-5-yl)-1,1'-biphenyl-4-yl]methyl]imidazol-5-carboxylic acid monohydrate (hereinafter referred to as "Compound F") was administered to the rats as follows.

Compound A Administration Group:

A physiological saline solution containing Compound A (0.005%) was instilled into both eyes 6 times a day for 14 days (one group consisting of 4 animals, 8 eyes). Compound B Administration Group:

A physiological saline solution containing Compound B (0.04%) was instilled into both eyes 6 times a day for 14 days (one group consisting of 4 animals, 8 eyes). Compound C Administration Group:
A physiological saline solution containing Compound C (0.5%) was instilled into both eyes 6 times a day for 14 days (one group consisting of 3 animals, 6 eyes).

Compound D Administration Group:

A phosphate-buffered saline solution containing Compound D (0.004%) was instilled into both eyes 6 times a day for 14 days (one group consisting of 4 animals, 8 eyes).

Compound E Administration Group:

A phosphate-buffered saline solution containing Compound E (0.004%) was instilled into both eyes 6 times a day for 14 days (one group consisting of 4 animals, 8 eyes).

Compound F Administration Group:

A phosphate-buffered saline solution containing Compound F (0.004%) was instilled into both eyes 6 times a day for 14 days (one group consisting of 4 animals, 8 eyes).

In a control group, physiological saline or phosphate-buffered saline was instilled into both eyes 6 times a day for 14 days (one group consisting of 4 animals, 8 eyes).

Fourteen days after the start of instillation, the damaged parts of the cornea were stained with fluorescein. For each of the upper, middle and lower parts of the cornea, the degree of fluorescein staining was evaluated by scoring according to the criteria shown below and the improvement ratio of corneal damage was calculated from the mean value of the total scores for each of the above-mentioned parts. Also for normal eyes, the mean value of the total scores for each of the above-mentioned parts was obtained.

(Evaluation Criteria)

0: No punctate staining
1: Scattered staining (punctate staining being separated)
2: Moderate staining (a part of punctate staining being adjacent)
3: Heavy staining (punctate staining being barely separated)

(Results)

By taking the mean value of the total scores for the control group (physiological saline or phosphate-buffered saline) as a standard (improvement ratio: 0%) and according to the equation shown below, the improvement ratios for the Compound A, B and C administration groups were calculated, respectively, which are shown in Table 1. The respective improvement ratios for the Compound D and E administration groups calculated in a similar manner are shown in Table 2, and the improvement ratio for the Compound F administration group is shown in Table 3. Incidentally, the mean value of the scores is a mean of those of 8 cases or 6 cases, respectively.

$$\text{Improvement ratio (\%)} = \{(\text{control}) - (\text{the present compound})\} / \text{damage degree} \times 100$$

$$\text{Damage degree} = (\text{control}) - (\text{normal eye})$$

[Table 1]

| Group | Mean value of scores | Improvement ratio (%) |
|---|---|---|
| Normal eye | 3.3 | |
| Control (physiological saline) administration group | 4.9 | |
| Compound A administration group (0.005%) | 3.9 | 62.5 |
| Compound B administration group (0.04%) | 3.9 | 62.5 |
| Compound C administration group (0.5%) | 3.5 | 87.5 |

[Table 2]

| Group | Mean value of scores | Improvement ratio (%) |
|---|---|---|
| Normal eye | 2.5 | |
| Control (phosphate-buffered saline) administration group | 4.6 | |
| Compound D administration group (0.004%) | 3.6 | 47.6 |
| Compound E administration group (0.004%) | 3.9 | 33.3 |

[Table 3]

| Group | Mean value of scores | Improvement ratio (%) |
|---|---|---|
| Normal eye | 2.2 | |
| Control (phosphate-buffered saline) administration group | 6.3 | |
| Compound F administration group (0.004%) | 3.6 | 65.9 |

(Discussion)

As apparent from the results of the above pharmacological test using rats (Tables 1 to 3), Compounds A to F significantly improved a corneal damage.

[Preparation Examples]

Hereinafter, representative preparation examples using Compounds A to F will be shown.

Preparation example 1

In 100 ml,

| | |
|---|---|
| Compound A | 10 mg |
| Sodium Chloride | 900 mg |
| Sterile purified water | q.s. |

By altering the amount of Compound A to be added, an eye drop at a concentration of 0.001% (w/v), 0.03% (w/v), 0.1% (w/v), 0.3% (w/v), 1.0% (w/v), or 3.0% (w/v) can be prepared.

Preparation example 2

In 100 ml,

| | |
|---|---|
| Compound B | 50 mg |
| Sodium Chloride | 800 mg |
| Disodium hydrogen phosphate | 100 mg |
| Sodium dihydrogen phosphate | q.s. |
| Sterile purified water | q.s. |

By altering the amount of Compound B to be added, an eye drop at a concentration of 0.002% (w/v), 0.01% (w/v), 0.25% (w/v), 1.25% (w/v), or 3% (w/v) can be prepared.

Preparation example 3

In 100 ml,

| | |
|---|---|
| Compound C | 100 mg |
| Sodium Chloride | 800 mg |
| Disodium hydrogen phosphate | 100 mg |
| Sodium dihydrogen phosphate | q.s. |
| Sterile purified water | q.s. |

By altering the amount of Compound C to be added, an eye drop at a concentration of 0.003% (w/v), 0.01% (w/v), 0.03% (w/v), 0.05% (w/v), 0.3% (w/v), 1% (w/v) or 3% (w/v) can be prepared.

Preparation example 4

In 100 ml,

| | |
|---|---|
| Compound D | 50 mg |
| Sodium Chloride | 900 mg |
| Sterile purified water | q.s. |

By altering the amount of Compound D to be added, an eye drop at a concentration of 0.002% (w/v), 0.01% (w/v), 0.25% (w/v), 1.25% (w/v), or 3% (w/v) can be prepared.

Preparation example 5

In 100 ml,

| | |
|---|---|
| Compound E | 100 mg |
| Sodium Chloride | 800 mg |
| Disodium hydrogen phosphate | 100 mg |
| Sodium dihydrogen phosphate | q.s. |
| Sterile purified water | q.s. |

By altering the amount of Compound E to be added, an eye drop at a concentration of 0.003% (w/v), 0.01% (w/v), 0.03% (w/v), 0.05% (w/v), 0.3% (w/v), 1% (w/v) or 3% (w/v) can be prepared.

Preparation example 6

In 100 ml,

| | |
|---|---|
| Compound F | 10 mg |
| Sodium Chloride | 800 mg |
| Disodium hydrogen phosphate | 100 mg |

(continued)

| | |
|---|---|
| Sodium dihydrogen phosphate | q.s. |
| Sterile purified water | q.s. |

By altering the amount of Compound F to be added, an eye drop at a concentration of 0.001% (w/v), 0.03% (w/v), 0.1% (w/v), 0.3% (w/v), 1.0% (w/v) or 3.0% (w/v) can be prepared.

Preparation example 7

In 100 g,

| | |
|---|---|
| Compound C | 0.3 g |
| Liquid paraffin | 10.0 g |
| White soft paraffin | q.s. |

By altering the amount of Compound C to be added, an ophthalmic ointment at a concentration of 1% (w/w) or 3% (w/w) can be prepared.

Preparation example 8

In 100 g,

| | |
|---|---|
| Compound F | 0.3 g |
| Liquid paraffin | 10.0 g |
| White soft paraffin | q.s. |

By altering the amount of Compound F to be added, an ophthalmic ointment at a concentration of 1% (w/w) or 3% (w/w) can be prepared.

## Claims

1. Use of a compound represented by the following general formula (1) or a salt thereof for manufacturing a therapeutic agent for a keratoconjunctival disorder:

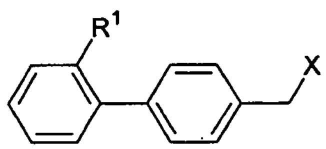

(1)

wherein X represents

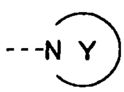

or

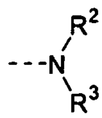

,

the ring Y represents a substituted or unsubstituted nitrogen-containing heterocyclic ring;
$R^1$ represents a carboxy group or a substituted or unsubstituted nitrogen-containing 5-membered heterocyclic ring; and

$R^2$ and $R^3$ may be the same or different and represent a hydrogen atom, a substituted or unsubstituted alkyl group or an unsubstituted alkylcarbonyl group.

**2.** The use according to claim 1, wherein in the general formula (1), X represents

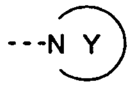,

the ring Y represents

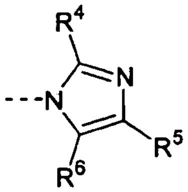, 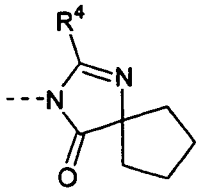 or 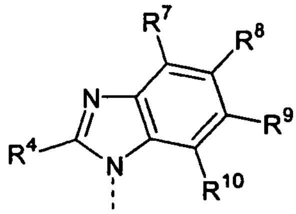,

$R^1$ represents a carboxy group,

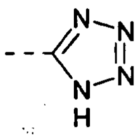 or 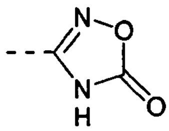,

$R^4$ represents a hydrogen atom, a hydroxy group, an alkoxy group or an alkyl group;
$R^5$ and $R^6$ may be the same or different and represent a halogen atom, a hydroxyalkyl group or an alkoxyalkyl group; and
$R^7$, $R^8$, $R^9$ and $R^{10}$ may be the same or different and represent a hydrogen atom, a hydroxy group, an alkoxy group or a carboxy group or an ester thereof.

**3.** The use according to claim 1 or 2, wherein in the general formula (1), X represents

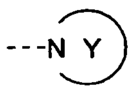,

the ring Y represents

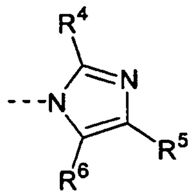,

$R^1$ represents a carboxy group or

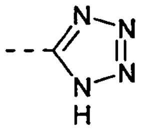

,

$R^4$ represents an alkoxy group or an alkyl group;
$R^5$ represents a halogen atom or a hydroxyalkyl group; and
$R^6$ represents a halogen atom, a hydroxyalkyl group or a carboxy group or an ester thereof.

**4.** The use according to claim 1 or 2, wherein in the general formula (1),
X represents

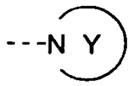

,

the ring Y represents

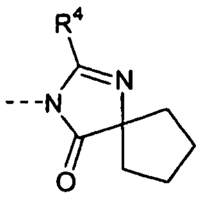

,

$R^1$ represents

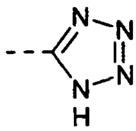

,

and
$R^4$ represents an alkyl group.

**5.** The use according to claim 1 or 2, wherein in the general formula (1),
X represents

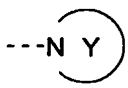

,

the ring Y represents

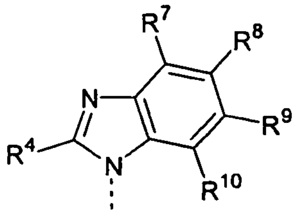

,

$R^1$ represents a carboxy group or

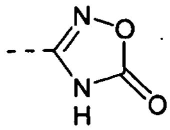

,

$R^4$ represents an alkoxy group or an alkyl group,
$R^7$ represents a hydrogen atom or an alkyl group,
$R^8$ represents a hydrogen atom,
$R^9$ represents a hydrogen atom or

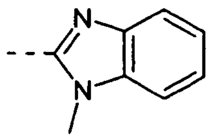

,

and
$R^{10}$ represents a hydrogen atom or a carboxy group or an ester thereof.

**6.** The use according to claim 3 or 5, wherein the ester of a carboxy group is

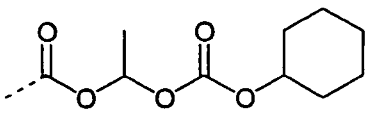

or

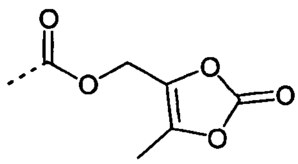

.

**7.** The use according to claim 1, wherein in the general formula (1),
X represents

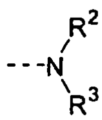

,

$R^1$ represents

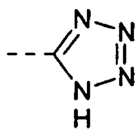

,

and
$R^2$ and $R^3$ may be the same or different and represent a hydrogen atom, a carboxyalkyl group or an alkylcarbonyl group.

**8.** The use according to claim 1 or 7, wherein in the general formula (1),
X represents

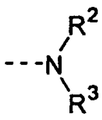

,

$R^1$ represents

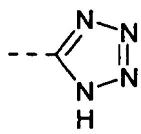

,

$R^2$ represents a carboxyalkyl group; and
$R^3$ represents an unsubstituted alkylcarbonyl group.

**9.** The use according to claim 1, wherein 4'-[[4-methyl-6-(1-methylbenzimidazol-2-yl)-2-n-propylbenzimidazol-1-yl]methyl]-1,1'-biphenyl-2-carboxylic acid, 2-n-butyl-4-spirocyclopentane-1-[[2'-(1H- tetrazol-5-yl)-1,1'-biphenyl-4-yl]methyl]-2-imidazolin-5-one, 2-butyl-4-chloro-5-hydroxymethyl-1-[[2'-(1H- tetrazol-5-yl)-1,1'-biphenyl-4-yl]methyl]imidazole, 2-ethoxy-1-[[2'-(1H-tetrazol-5-yl)-1,1'-biphenyl-4-yl]methyl]-1H-benzimidazol-7-carboxylic acid, 1-(cyclohexyloxycarbonyloxy)ethyl 2-ethoxy-1-[[2'-(1H-tetrazol-5-yl)-1,1'-biphenyl-4-yl]methyl]-1H-benzimidazol-7-carboxylate, N-[[2'-(1H-tetrazol-5-yl)-1,1'- biphenyl-4-yl]methyl]-N-valeryl-L-valine, 4-(1-hydroxy-1-methylethyl)-2-n-propyl-1-[[2'-(1H-tetrazol-5-yl)-1,1'-biphenyl-4-yl]methyl]imidazol-5-carboxylic acid or (5-methyl-2-oxo-1,3-dioxol-4-yl)methyl 4-(1- hydroxy-1-methylethyl)-2-n-propyl-1-[[2'-(1H-tetrazol-5-yl)-1,1'-biphenyl-4-yl]methyl]imidazol-5-carboxylate, or a salt thereof is contained in the therapeutic agent as an active ingredient.

**10.** The use according to any one of claims 1 to 9, wherein the keratoconjunctival disorder is dry eyes, corneal ulcer, keratitis, conjunctivitis, superficial punctate keratopathy, corneal epithelial defects, conjunctival epithelial defects, keratoconjunctivitis sicca, superior limbic keratoconjunctivitis or filamentary keratitis.

**11.** The use according to any one of claims 1 to 10, wherein the dosage form is an eye drop or an ophthalmic ointment.

## Patentansprüche

**1.** Verwendung einer Verbindung, dargestellt durch die folgende allgemeine Formel (1), oder einem Salz davon zur Herstellung eines therapeutischen Mittels für eine keratokonjunktivale Störung:

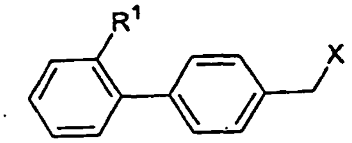 (1)

worin X

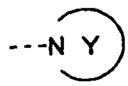 oder 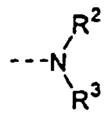

darstellt,
wobei der Ring Y einen substituierten oder unsubstituierten stickstoffhaltigen heterocyclischen Ring darstellt,
$R^1$ eine Carboxygruppe oder einen substituierten oder unsubstituierten stickstoffhaltigen 5-gliedrigen heterocyclischen Ring darstellt,
$R^2$ und $R^3$ gleich oder unterschiedlich sein können und ein Wasserstoffatom, eine substituierte oder unsubstituierte Alkylgruppe oder eine unsubstituierte Alkylcarbonylgruppe darstellen.

**2.** Verwendung gemäß Anspruch 1, wobei in der allgemeinen Formel (1)

X 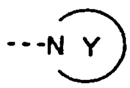

darstellt, der Ring Y

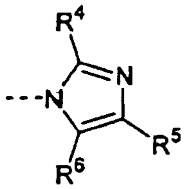, 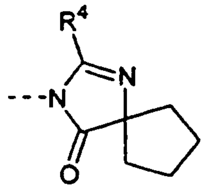 oder 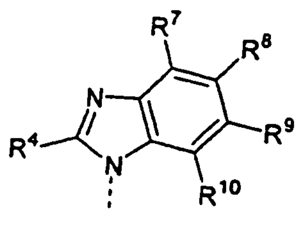

darstellt,
$R^1$ eine Carboxygruppe

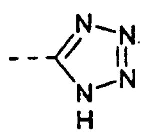 oder 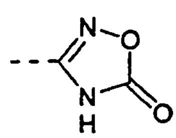

darstellt,
$R^4$ ein Wasserstoffatom, eine Hydroxygruppe, eine Alkoxygruppe oder eine Alkylgruppe darstellt,
$R^5$ und $R^6$ gleich oder unterschiedlich sein können und ein Halogenatom, eine Hydroxyalkylgruppe oder eine Alkoxyalkylgruppe darstellen, und

$R^7$, $R^8$, $R^9$ und $R^{10}$ gleich oder unterschiedlich sein können und ein Wasserstoffatom, eine Hydroxygruppe, eine Alkoxygruppe oder eine Carboxygruppe oder einen Ester davon darstellen.

**3.** Verwendung gemäß Anspruch 1 oder 2, wobei in der allgemeinen Formel (1)
X

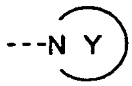

darstellt,
der Ring Y

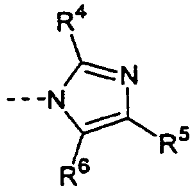

darstellt, $R^1$ eine Carboxygruppe oder

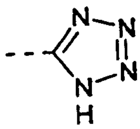

darstellt,
$R^4$ eine Alkoxygruppe oder eine Alkylgruppe darstellt,
$R^5$ ein Halogenatom oder eine Hydroxyalkylgruppe darstellt und
$R^6$ ein Halogenatom, eine Hydroxyalkylgruppe oder eine Carboxygruppe oder einen Ester davon darstellt.

**4.** Verwendung gemäß Anspruch 1 oder 2, wobei in der allgemeinen Formel (1)
X

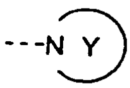

darstellt,
der Ring Y

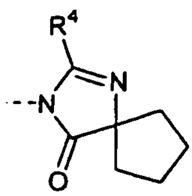

darstellt,

$R^1$

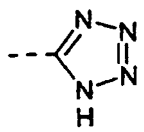

darstellt und
$R^4$ eine Alkylgruppe darstellt.

**5.** Verwendung gemäß Anspruch 1 oder 2, wobei in der allgemeinen Formel (1)
X

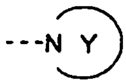

darstellt,
der Ring Y

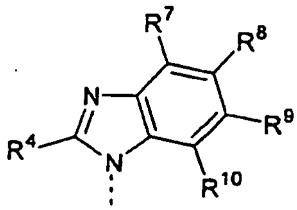

darstellt,
$R^1$ eine Carboxygruppe oder

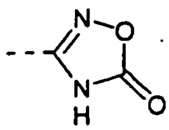

darstellt,
$R^4$ eine Alkoxygruppe oder eine Alkylgruppe darstellt,
$R^7$ ein Wasserstoffatom oder eine Alkylgruppe darstellt,
$R^8$ ein Wasserstoffatom darstellt,
$R^9$ ein Wasserstoffatom oder

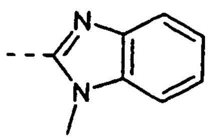

darstellt und
$R^{10}$ ein Wasserstoffatom oder eine Carboxygruppe oder einen Ester davon darstellt.

**6.** Verwendung gemäß Anspruch 3 oder 5, wobei der Ester einer Carboxygruppe

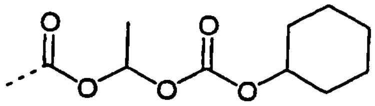 oder 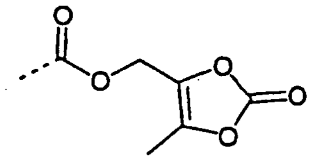

ist.

**7.** Verwendung gemäß Anspruch 1, wobei in der allgemeinen Formel (1)
X

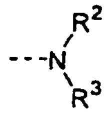

darstellt,
$R^1$

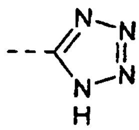

darstellt und
$R^2$ und $R^3$ gleich oder unterschiedlich sein können und ein Wasserstoffatom, eine Carboxyalkylgruppe oder eine Alkylcarbonylgruppe darstellen.

**8.** Verwendung gemäß Anspruch 1 oder 7, wobei in der allgemeinen Formel (1)
X

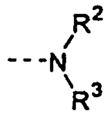

darstellt,
$R^1$

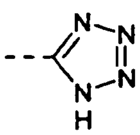

darstellt, $R^2$ eine Carboxyalkylgruppe darstellt und $R^3$ eine unsubstituierte Alkylcarbonylgruppe darstellt.

**9.** Verwendung gemäß Anspruch 1, wobei 4'-[[4-Methyl-6-(1-methylbenzimidazol-2-yl)-2-n-propylbenzimidazol-1-yl] methyl]-1,1'-biphenyl-2-carboxylsäure, 2-n-Butyl-4-spirocyclopentan-1-[[2'-(1H-tetrazol)-5-yl)-1,1'-biphenyl-4-yl] methyl]-2-imidazolin-5-on, 2-Butyl-4-chlor-5-hydroxymethyl-1-[[2'-(1H-tetrazol-5-yl)-1,1'biphenyl-4-yl]methyl]imidazol, 2-Ethoxy-1-[[2'-(1H-tetrazol-5-yl)-1,1'-biphenyl-4-yl]methyl]-1H-benzimidazol-7-carboxylsäure, 1-(Cyclohexyloxycarbonyloxy)ethyl 2-ethoxy-1-[[2'-(1H-tetrazol-5-yl)-1,1'-biphenyl-4-yl]methyl]-1H-benzimidazol-7-carboxylat, N-[[2'-(1H-tetrazol-5-yl)-1,1'-biphenyl-4-yl]methyl]-N-valeryl-L-valin, 4-(1-Hydroxy-1-methylethyl)-2-n-propyl-1-[[2'-(1H-tetrazol-5-yl)-1,1'-biphenyl-4-yl]methyl]imidazol-5-carbonsäure oder (5-Methyl-2-oxo-1,3-dioxol-4-yl)methyl 4-(1-hydroxy-1-methylethyl)-2-n-propyl-1-[[2'-(1H-tetrazol-5-yl)-1,1'-biphenyl-4-yl)methyl]imidazol-5-carboxylat oder ein Salz davon in dem therapeutischen Mittel als ein aktiver Bestandteil enthalten ist.

**10.** Verwendung gemäß einem der Ansprüche 1 bis 9, wobei die keratokonjunktivale Störung trockene Augen, ein Hornhautgeschwür, Keratitis, Konjunktivitis, Thygeson Keratitis, nicht heilendes Hornhautulcus, Bindehaut-Epithel-Defekte, Keratoconjunctivitis sicca, superior limbic keratoconjunctivitis oder filiforme Keratitis ist.

**11.** Verwendung gemäß einem der Ansprüche 1 bis 10, wobei die Verabreichungsform Augentropfen oder eine ophthalmische Salbe ist.

## Revendications

**1.** Utilisation d'un composé représenté par la formule générale suivante (1) ou d'un sel de celui-ci pour la préparation d'un agent thérapeutique pour un désordre kérato-conjonctival :

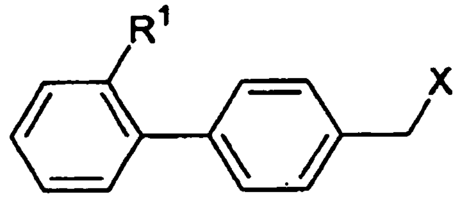

(1)

où X représente :

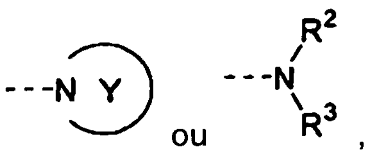

le cycle Y représente un hétérocycle contenant de l'azote, substitué ou non substitué ;
$R^1$ représente un radical carboxyle ou un hétérocycle à 5 membres, contenant de l'azote, substitué ou non substitué ; et
$R^2$ et $R^3$ peuvent être identiques ou différents et représentent l'atome d'hydrogène, un radical alkyle substitué ou non substitué ou un radical alkylcarbonyle non substitué.

**2.** Utilisation selon la revendication 1, où dans la formule générale (1), X représente :

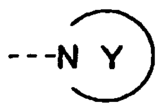

le cycle Y représente :

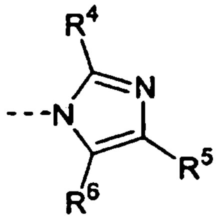,

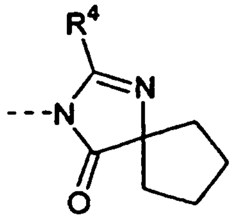

ou

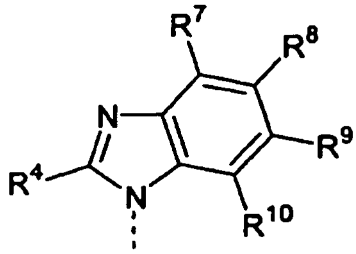

$R^1$ représente un radical carboxy,

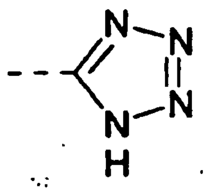

ou

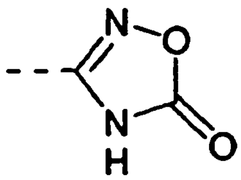,

$R^4$ représente l'atome d'hydrogène, le radical hydroxyle, un radical alcoxy ou un radical alkyle ; $R^5$ et $R^6$ peuvent être identiques ou différents et représentent un atome d'halogène, un radical hydroxyalkyle ou un radical alcoxyalkyle, et $R^7$, $R^8$, $R^9$ et $R^{10}$ peuvent être identiques ou différents et représentent l'atome d'hydrogène, le radical hydroxyle, un radical alcoxy ou un radical carboxy ou un ester de celui-ci.

**3.** Utilisation selon la revendication 1 ou 2, où dans la formule générale (1), X représente :

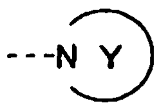

le cycle Y représente :

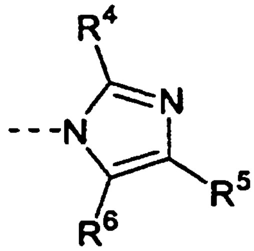,

$R^1$ représente un radical carboxy ou

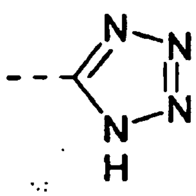

$R^4$ représente un radical alcoxy ou un radical alkyle ;
$R^5$ représente un atome d'halogène ou un radical hydroxyalkyle, et
$R^6$ représente un atome d'halogène, un radical hydroxyalkyle ou un radical carboxy ou un ester de celui-ci.

**4.** Utilisation selon la revendication 1 ou 2, où dans la formule générale (1), X représente :

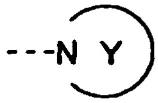

le cycle Y représente :

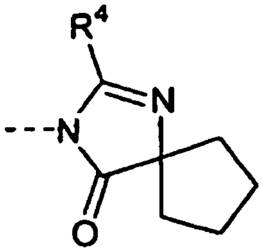

$R^1$ représente :

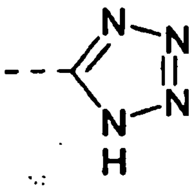

,

et
$R^4$ représente un radical alkyle.

**5.** Utilisation selon la revendication 1 ou 2, où dans la formule générale (1),
X représente :

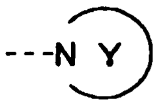

le cycle Y représente :

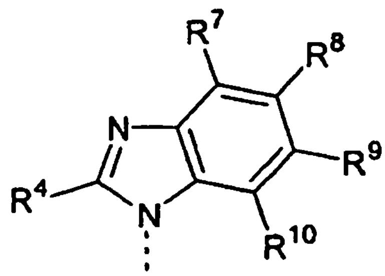

,

$R^1$ représente un radical carboxy ou

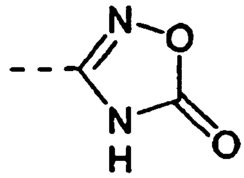

,

$R^4$ représente un radical alcoxy ou un radical alkyle,
$R^7$ représente l'atome d'hydrogène ou un radical alkyle,
$R^8$ représente l'atome d'hydrogène,
$R^9$ représente l'atome d'hydrogène, ou

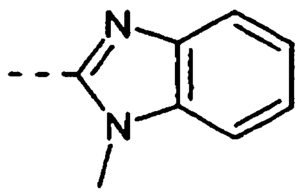

,

et
$R^{10}$ représente l'atome d'hydrogène ou un radical carboxy ou un ester de celui-ci.

**6.** Utilisation selon la revendication 3 ou 5, où l'ester d'un radical carboxy est

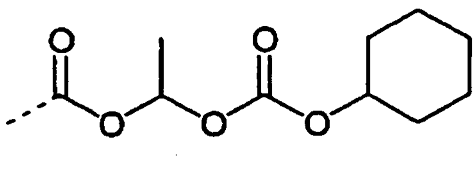

ou

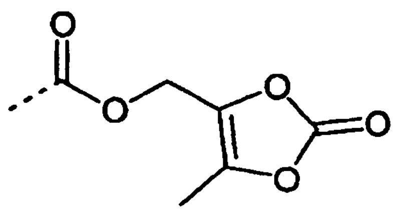

.

**7.** Utilisation selon la revendication 1, où dans la formule générale (I),
X représente :

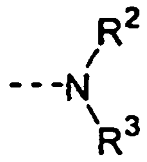

$R^1$ représente :

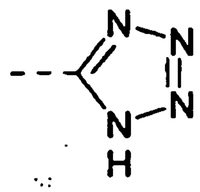

et
$R^2$ et $R^3$ peuvent être identiques ou différents et représentent un atome d'hydrogène, un radical carboxyalkyle ou un radical alkylcarbonyle.

**8.** Utilisation selon la revendication 1 ou 7, où dans la formule générale (I),
X représente :

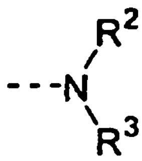

$R^1$ représente :

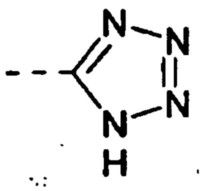

$R^2$ représente un radical carboxyalkyle, et
$R^3$ représente un radical alkylcarbonyle non substitué.

**9.** Utilisation selon la revendication 1, où l'agent thérapeutique contient en tant qu'ingrédient actif, l'acide 4'-[[4-méthyl-6-(1-méthylbenzimidazol-2-yl)-2-n-propylbenzimidazol-1-yl]méthyl]-1,1'-biphényl-2-carboxylique, la 2-n-butyl-4-spirocyclopentane-1-[[2'-(1H-tétrazol-5-yl)-1,1'-biphényl-4-yl]méthyl]-2-imidazolin-5-one, le 2-butyl-4-chloro-5-hydroxyméthyl-1-[[2'-(1H-tétrazol-5-yl)-1,1' -biphényl-4-yl]méthyl]imidazole, l'acide 2-éthoxy-1-[[2'-(1H-tétrazol-5-yl)-1,1'-biphényl-4-yl]méthyl]-1H-benzimidazol-7-carboxylique, le 2-éthoxy-1-[[2'-(1H-tétrazol-5-yl)-1,1'-biphényl-4-yl]méthyl]-1H-benzimidazol-7-carboxylate de 1-(cyclohexyloxycarbonyloxy)éthyle, la N-[[2'-(1H-tétrazol-5-yl)-1,1'-biphényl-4-yl]méthyl]-N-valéryl-L-valine, l'acide 4-(1-hydroxy-1-méthyléthyl)-2-n-propyl-1-[[2'-(1H-tétrazol-5-yl)-1,1'-biphényl-4-yl]méthyl]imidazol-5-carboxylique ou le 4-(1-hydroxy-1-méthyléthyl)-2-n-propyl-1-[[2'-(1H-tétrazol-5-yl)-1,1'-biphényl-4-yl]méthyl]imidazol-5-carboxylate de (5-méthyl-2-oxo-1,3-dioxol-4-yl)méthyle, ou un sel de ceux-ci.

**10.** Utilisation selon l'une quelconque des revendications 1 à 9, où le désordre kérato-conjonctival est la sécheresse oculaire, l'ulcère de la cornée, la kératite, la conjonctivite, la kératopathie ponctuée superficielle, des défauts épithéliaux cornéens, des défauts épithéliaux de la conjonctive, la kérato-conjonctivite sicca, la kérato-conjonctivite limbique supérieure ou la kératite filamenteuse.

**11.** Utilisation selon l'une quelconque des revendications 1 à 10, où la forme galénique est des gouttes oculaires ou un onguent ophtalmique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2709225 B
- JP 2868313 B
- JP 5029351 B
- JP 2853611 B
- JP 2514282 B
- JP 2749458 B
- JP 7025738 B
- JP 2645962 B
- JP 3465215 B


### Non-patent literature cited in the description


- *Japanese Review of Clinical Ophthalmology,* 1992, vol. 46, 738-743
- *Ophthalmic Surgery,* 1992, vol. 5, 719-727
- **Fujihara et al.** *Invest. Ophthalmol. Vis. Sci.,* 2001, vol. 42 (1), 96-100
- **Murakami et al.** *Journal of the eye,* 2004, vol. 21 (1), 87-90